Europäisches Patentamt

European Patent Office

Office européen des brevets

(11) Publication number: **0 184 399**
**A2**

## (12) EUROPEAN PATENT APPLICATION

(21) Application number: **85308703.9**

(22) Date of filing: **29.11.85**

(51) Int. Cl.⁴: **A 61 B 1/00**
**A 61 M 25/00, A 61 B 1/30**

(30) Priority: **03.12.84 US 677558**

(43) Date of publication of application:
**11.06.86 Bulletin 86/24**

(84) Designated Contracting States:
**DE FR GB IT SE**

(71) Applicant: **Patel, Jayendrakumar I.**
**502 Rector Street**
**Valdese North Carolina 28690(US)**

(72) Inventor: **Patel, Jayendrakumar I.**
**502 Rector Street**
**Valdese North Carolina 28690(US)**

(74) Representative: **Weston, Robert Dale et al,**
**c/o PHILLIPS & LEIGH 7 Staple Inn**
**Holborn London WC1V 7QF(GB)**

(54) **Endoscope.**

(57) An endoscope comprising a flexible tube (10) having a proximate end portion (27) in communication with a control unit (8), a body portion and a distal end portion (10'), the tube containing light transmitting means (20) whereby objects outside the free end of the distal end portion may be illuminated and viewed through the control unit; a further flexible outer tube (9) being co-axially slideable about the body and distal end portion (10') of the inner tube (10) is provided with means (11) guarding the distal end of the inner tube whilst allowing relative incremental axial movement of the inner and outer tubes without the risk of tissue being trapped between them.

FIG.2

EP 0 184 399 A2

0184399

1.

ENDOSCOPE

The invention relates to an endoscope used to view the internal sidewalls of the organs of a human or animal body from a point outside of such body. More particularly, the invention is an endoscope that has an elongated tube member used to be inserted through linear, nonlinear, curved and/or looped passages within a human and or animal body so that the sidewalls of such body can be visually observed through a viewing apparatus located outside of the body. One of the drawbacks encountered in the use of prior art endoscopes, especially in humans, is the pain and discomfort experienced by the patient. This, coupled with the high degree of skill a medical practitioner must have in order to insert and manipulate prior art endoscopes into a human cavity, limits their widespread use. This is unfortunate because deep penetration into a human organ is possible with an endoscope and more often than not the medical practitioner desires (for professional and ethical reasons) to make an examination of the deepest regions of the organ in question by advancing the endoscope to its physical limits.

In many cases, it is not only desirable but professionally required that a medical practitioner visually examine the internal organs or passages of a human and/or animal body for diagnosis, biopsy, or

2.

for removal of obstructions, foreign bodies or stones without cutting open the patient and the organ to be examined. One method of visually examining the internal organs of a patient without surgery is to insert the flexible fiber optic portion of an endoscope into the body through a natural or a surgically prepared opening. The distal (inspection) portion of the instrument (in optical communication with a control unit) illuminates the area and also permits inspection through a viewing device from outside of the body under examination. Endoscopes are well known in the prior art and details of the construction of a flexible fiber optic endoscope are set forth in U.S. Patents 3,610,231; 3,788,304; 4,273,111 and 4,292,961, the disclosures of which are herein set forth as if faithfully reproduced.

Notwithstanding the fact that endoscopes are well known to the medical profession, experienced operators often experience problems in properly placing the distal end of the instrument in the deep areas of a human or animal body. Body passages, often nonlinear, are made of soft, stretchable, flexible, foldable, pressure and pain sensitive tissue. Invariably they have bends or loops which are difficult to negotiate without causing pain to the patient. U.S. Patents

3.

4,292,961 and 4,366,810 both recognise these difficulties and disclose complex apparatus and methods in an attempt to solve these problems. They are complex,expensive,difficult to manipulate and require a high degree of skill to use. It is towards the solution of the above stated problems that the instant invention is directed.

According to the present invention an endoscope comprises a flexible tube having a proximate end portion in communication with a control unit, a body portion and a distal end portion, the tube containing light transmitting means whereby objects outside the free end of the distal end portion may be illuminated and viewed through the control unit; a further flexible outer tube is co-axially slideable about the body and distal end portions of the inner tube and is provided with means guarding the distal end of the inner tube whilst allowing relative incremental axial movements of the inner and outer tubes without the risk of tissue being trapped between them. The endoscope is introduced by an operator first inserting the distal end portion of the inner tube a short distance into an orifice; then, holding still the proximate end portion of the inner tube, the outer tube is slid an equally short distance in over the inner tube; the proximate end portion of the outer tube is then held still

4.

whilst the inner tube is advanced another short distance; and the action is repeated in an "inchworming" effect until the endoscope has been inserted to the required depth.

In a first embodiment of the present invention, the guard means comprise expansion and contraction means attached between the distal end of the outer tube and a point near the distal end portion of the inner tube so that the means can expand and contract axially of the inner and outer tubes. In a variant, further expansion and contraction means are attached between the proximate end of the outer tube and the proximate end portion of the inner tube.

In a second embodiment of the present invention, the guard means comprise a tubular shield co-axially encompassing the distal end portion of the outer tube, the distal end of the tubular shield being attached to the inner tube adjacent the distal end portion thereof; the outer tube being axially reciprocable between the inner tube and the tubular shield.

Both these embodiments take advantage of the fact that it is inherent with most organs into which the tube of an endoscope is inserted to clamp down onto the tube once insertion begins. Such clamping propensity onto the outer tube aids and assists the

5.

advancement of the distal portion of the inner tube by helping to hold steady the outer tube.

In a third embodiment of the present invention, the guard means comprise a radially outwardly flared, bell-shaped end to the distal end portion of the outer tube.

In any of the embodiments, wall surfaces of the tubes may be provided with undulations to reduce friction.

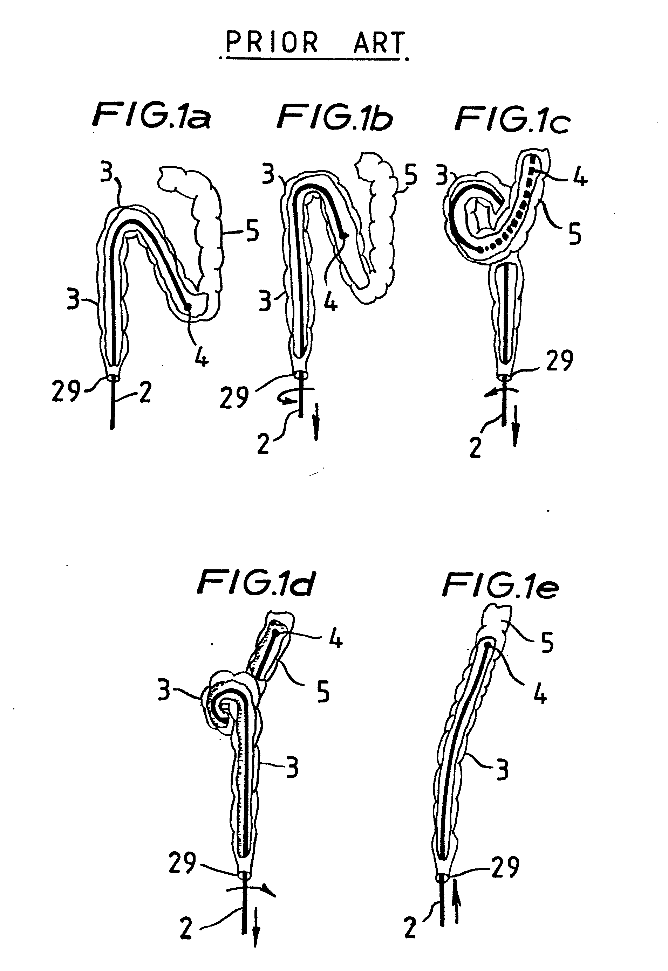

Any embodiment of the present invention overcomes the problem inherent in prior endoscopes, i.e., even though the distal end of a prior art endoscope can be pushed towards the desired direction the force or push applied to advance the endoscope is not necessarily transmitted to the distal end when the distal end has gone beyond a loop or a curved passageway. For example, but not by way of limitation, insertion of a prior art colonscope (a variety of endoscope) into the deeper areas of the human colon poses many difficulties due to the anatomy of the colon. The human colon has curves or bends (known as loops) and is made up of soft, stretchable, foldable, very pain sensitive tissues. In addition, it is affixed to the abdomen in some places and free in others. After the distal end of the colonscope passes through the initial curve, which is rather close to the rectum, further pushing of the scope results in enlargement of the curve rather than the desirable advancement of the distal portion. This results in the stretching of the colon and is very painful to the patient. One method, known to the prior art, of facilitating the further advancement of a colonscope by such a procedure is illustrated by reference to Figures 1(a), 1(b), 1(c), 1(d) and 1(e), where a technique (not necessarily the only technique) for straightening out a colon loop is shown. An attempt to advance an endoscope through this first loop only results in its enlargement; therefore, it must be first straightened out. While there are various techniques to "straighten out a colon loop", one that is shown in the previously referenced figure numerals is illustrative. Distal tube portion 4 of the endoscope is inserted through anus 29 into the sigmoid portion 3 of the colon and forms a long loop with an acute angle at the junction of the sigmoid 3 and descending colon 5. The next step is to withdraw the instrument to the mid-sigmoid colon position as shown in Figure 1(b), leaving a free segment of the sigmoid portion of the colon 3 for subsequent rotation. Subsequently the operator rotates counter clockwise the sigmoid loop producing an alfa loop pattern as shown in Figure 1(c)

7.

and then removes the alfa loop by rotating the endoscope tube portion clockwise and simultaneously withdrawing the instrument (Figure 1(d)), thereby producing a straightened sigmoid colon as shown in Figure 1(e). The ability to successfully perform such endoscopy procedures with expertise and minimal patient discomfort depends largely upon the degree of technical skill and experience of the operator. In order to attain these skills, there is, of course, no substitute for actual "hands on" patient experience. The finesse required to perform these manuevers can only be achieved by repetitive training and unfortunately with a great deal of discomfort and pain to the patient.

By use of any one of the embodiments of the disclosed invention, the painful experience that the patient must be put through and the training required of the practitioner as was the case with prior art endoscopes is not necessary because the colon loops do not need to be straightened out. The endoscope of the present invention (all three embodiments) using the recommended procedures will follow the curves of the colon as they appear with minimal pain and discomfort to the patient.

The above and other features of the present invention are illustrated by way of example in the Drawings, wherein:-

Figure 2 is an oblique external view of a first embodiment of an endoscope in accordance with the present invention;

Figure 3 is a similar view of a variant of the endoscope of Figure 2;

Figures 4a and 4b are oblique partial sections of the distal and proximate end portions of the endoscope of Figure 3, illustrating expanded and contracted conditions;

Figure 5 is an oblique view of a second embodiment of an endoscope in accordance with the present invention;

Figure 6a is a similar view of a third embodiment of the present invention;

Figure 6b is a section on the line 6b-6b of Figure 6a;

Figures 7a and 7b are oblique partial sections of the distal end portion of the endoscope of Figure 5, illustrating the reciprocal axial movement of the outer tube within the shield;

Figures 8a and 8b are oblique partial sections of the distal end portion of the endoscope of Figure 6a, illustrating the reciprocal axial movement of the distal end portion of the inner tube within the bell-mouthed distal end of the outer tube;

Figures 9a, 9b and 9c are graphic illustrations of the endoscope of Figure 3 showing how it is inserted

9.

and advanced in a human colon and through the first encountered loop; and

Figures 10a, 10b and 10c are axial sections of inner and outer tubes illustrating various provisions of wall surface undulations.

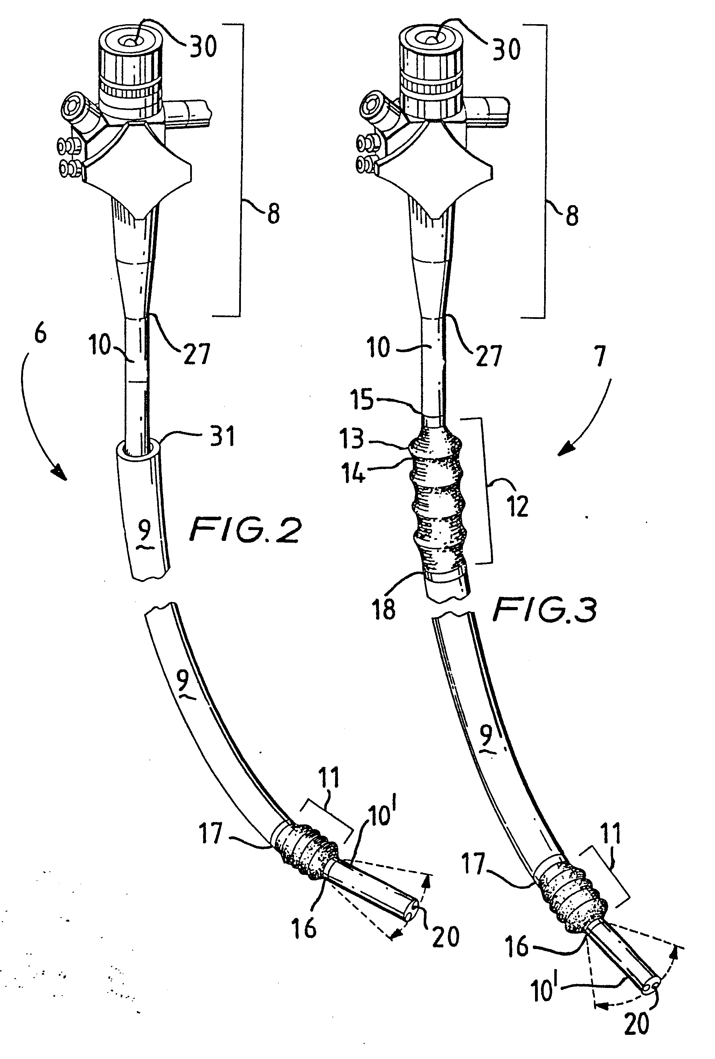

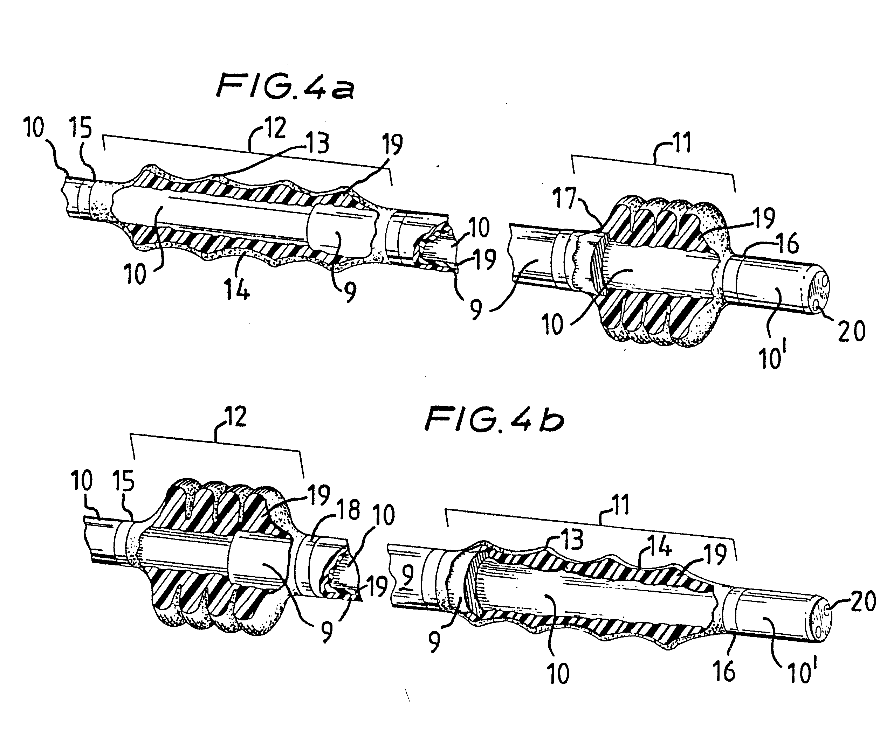

Referring to Figure 2, there is shown an endoscope 6 being a first embodiment of the invention. Control unit 8, which is of prior art design and contains the customary view piece 30 with well known control knobs the description of which serves no part of the instant invention (see description of same in previously identified prior art patents), is optically connected to a first or inner elongate flexible tube 10, at junction 27. Inner tube member 10 contains prior art optical means (not shown) and is composed of a proximate end portion (adjacent element 27), and a distal portion 10' connected by a body portion (that portion of element 10 between the distal and proximate end portions). Distal end portion 10' is movable up, down, left and right and contains viewing and illuminating elements 20; such as fiber optic means (not shown) in the body and proximate portions of flexible tube 10, through viewer means 30 and a light source in control unit 8. Movement of distal portion 10' is accomplished by rotating appropriate knobs that control cables (not shown) in the interior of tube 10 and 10' in a known prior art manner.
Inner tube 10 is co-axially nested inside and circumscribed
by a flexible outer tube 9. Outer tube 9 has one free
proximate end 31 spaced apart from control unit 8 and
a distal end portion fixedly attached to a distal accordion
or bellows shaped means 11 at point 17. Distal bellows
or accordion shaped means 11 is also affixed to inner
tube 10 at a point 16 adjacent to the distal portion 10'.
Outer tube 9 is adapted to reciprocate co-axially around
and axially of inner tube 10; this reciprocation taking
place to the extent that the bellows 11 (shown in a
compressed state in Figure 2 can be extended and compressed
in the fashion as shown by Figures 4(a) and 4(b). In between
the inner surface of outer tube 9 and bellows 11 and the
outer surface of inner tube 10 may be some suitable lubricant,
much in the manner as shown in Figure 4(a). The internal
optical and control cable construction of inner tube 10
in combination with control unit 8 is common for this
and all hereinafter described embodiments.

A variant to this first embodiment is shown by
endoscope 7 of Figure 3. This variant includes the
same elements described in respect of first endoscope 6,
and in addition thereto contains a second, proximate
bellows or accordion means 12. It will be noted that
proximate bellows 12 (like bellows 11) has plurality of ridges 13 and valleys 14 to form the accordion or bellows type structure and is, like distal bellows 11, adapted to expand and contract upon movement of outer tube 9 while holding stationary inner tube 10 and vice versa. Proximate bellows 12 is affixed to inner tube 10 at the point 15 (near the proximate end portion of inner tube 10) and is also affixed to outer tube 9 at point 18, i.e. its proximate end portion. The space delimited by and between tubes 9 and 10 and bellows 11 and 12, as more fully described, may be filled with a suitable grease or lubricant.

The basic operation of endoscopes 6 and 7 is as follows: holding inner tube 10 stationary, outer tube 9 is advanced thereover to the extent that distal bellows 11 can be compressed and/or proximate bellows 12 can be extended. Bellows 11 and 12 are usually of the same construction and length and therefore they cooperate with one another in extending and contracting. Once outer tube 9 has been advanced as far as distal bellows 11 will permit and/or proximate bellows 12 can be extended, then outer tube 9 is held stationary while inner tube 10 is advanced a distance that either the compression of proximate bellows 12 will permit or the extension of distal bellows 11 will allow. This "inchworm" expansion and contraction is repeated over

12.

and over and over again and by this repetition, the distal end 10' of inner tube 10 is advanced a short distance each expansion and contraction until it finally reaches its destination point within the organ sought to be examined.

Reference is made to Figures 4(a) and 4(b) for a more detailed and graphic representation of this expansion and contraction characteristic of bellows 11 and 12 of the endoscope 7. If distal bellows 11 is compressed as per the manner previously described, the proximate bellows 12 is extended and vice versa. The space delimited by the inner surface of bellows 11 and 12, outer surface of inner tube 10 and the inner surface of outer tube 9 may be filled with a prior art lubricant such as petrolatum or mineral oil 19. When outer tube 9 is held stationary and inner tube 10 (of which one terminal portion contains distal member 10') is pushed forward, extended proximate means 12 of Figure 4(a) will then compress to the compressed configuration of Figure 4(b) and the compressed distal bellows 11 of Figure 4(a) will be extended, as shown in the Figure 4(b).

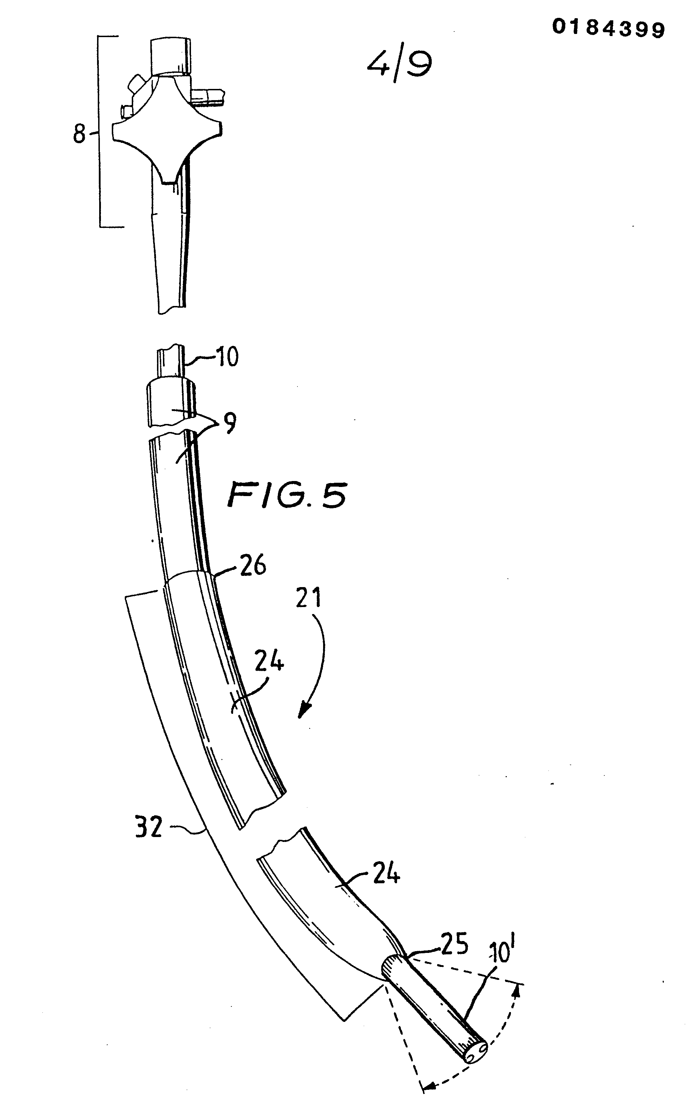

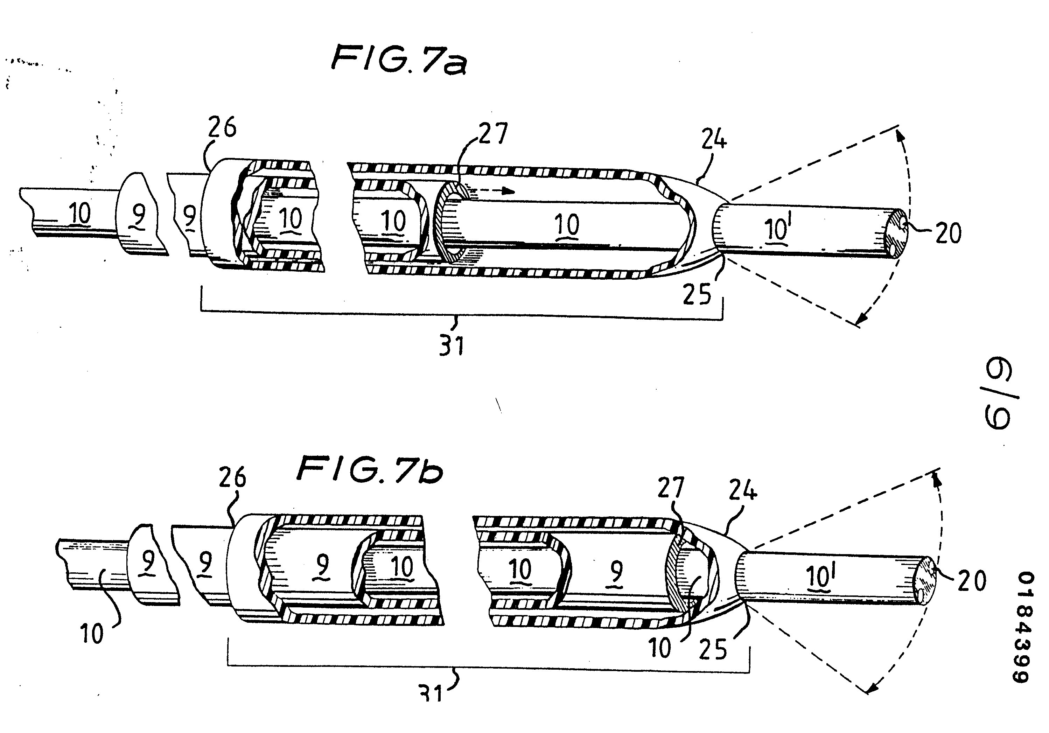

A second embodiment of an endoscope 21 is shown by Figure 5 to contain a control unit 8, joined to inner tube 10 at its proximate end, and is composed of a body portion, a tubular shield 32 and distal portion 10'. Outer tube 9 circumscribes and has nested therein inner tube 10; the outer tube has a body portion connecting its proximate end portion (portion nearest control unit 8) and its distal end portion terminating in free distal end 28. Shield 32 is composed of a tubular member 24, one end of which is tapered and fixed to inner tube 10 adjacent the distal portion 10 thereof. The sidewall of tubular shield 24 (see Figures 7(a) and 7(b))extends coaxially along the axis of inner tube 10 to the terminal mouth 26 of tubular shield 24. Distal free end 28 of outer tube 9 can be spaced apart from the junction 25 between tubular shield 24 and inner tube 10. By comparing the location of the distal free end 28 of the outer tube 9 of Figure 7(a) with the location of the distal free end 27 in Figure 7(b), one can grasp the teaching that outer tube 9 can be reciprocated from a first to a second position within the confines of the tubular shield 24. A stop means (not shown) may be placed on the outer surface of outer tube 9 and inner surface of tubular shield 24 near its mouth 26 to keep the distal free end 28 of outer tube 9 within the confines of tubular shield 24.

Using the endoscope 21 of Figure 5 and the

14.

teachings of its reciprocating manipulation as shown in Figures 7(a) and 7(b), one can readily realize that after insertion into an organ sought to be examined, by holding stationary outer tube 9, inner tube 10 can be advanced to that position as shown in Figure 7(a). Then, by holding stationary inner tube 10, outer tube 9 can be advanced to that position as shown in Figure 7(b). Repetition of such procedure, much like the "inchworm" manner as previously described for the first embodiment of the invention, causes the distal portion 10' to be advanced along loops and curves and twists and turns of the organ cavity of an animal or human to reach the deepest regions thereof, without having to straighten out any loops or curves as was the case with prior art devices, and without trapping organ matter (tissue) between the tubes 9 and 10.

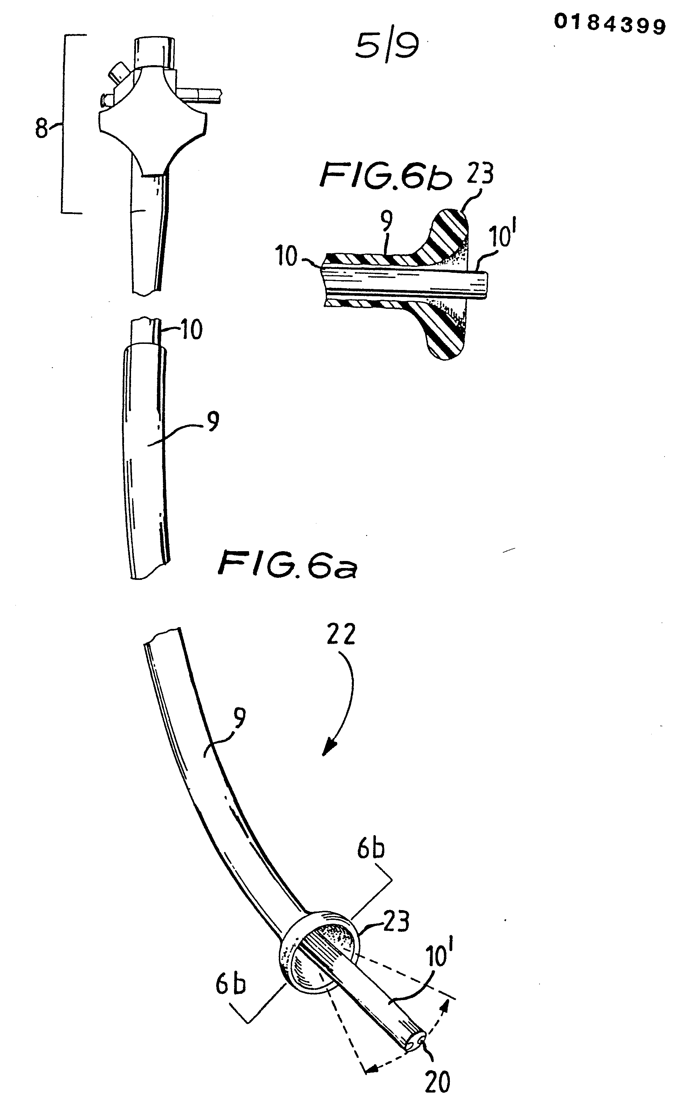

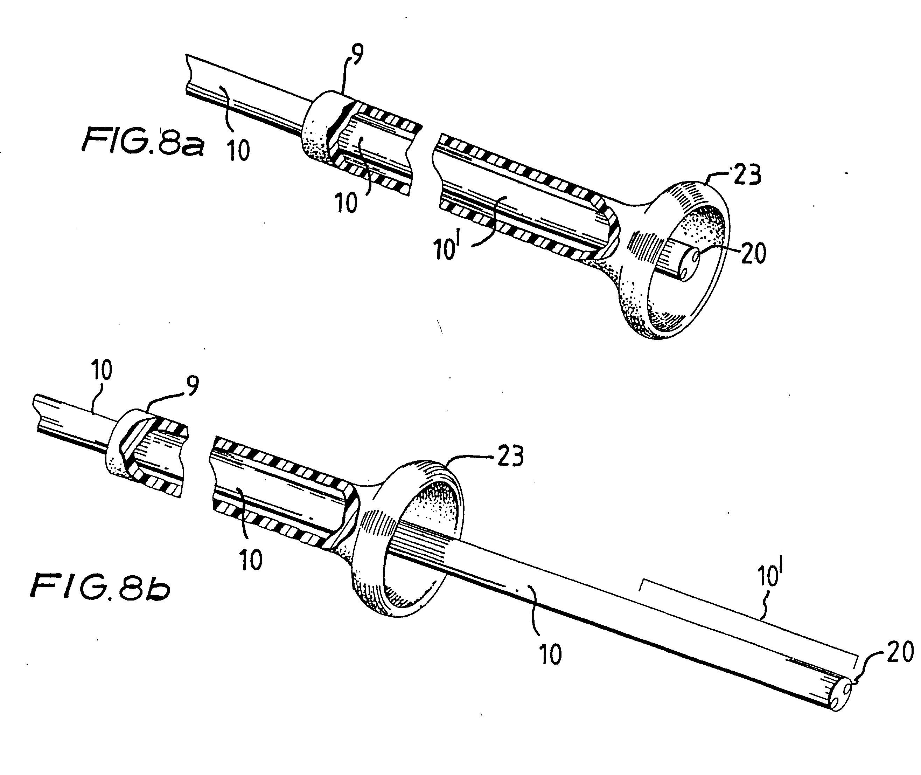

A third embodiment is shown by endoscope 22 in Figures 6(a) and 6(b). Like previously described embodiments, it is composed of a control unit 8, inner tube 10 having a body portion connected to proximate and distal end portions (the proximate end portion is connected to control unit 8) and an outer tube 9. Inner tube 10 is coaxially nested inside of outer tube 9. Outer tube 9 has an outward flaring distal end portion 23 and a proximate free end portion. In cross section, the distal end portion looks much like a bell, a more detailed description being shown in Figures 6(b), 8(a) and 8(b). In use, inner tube 10 is inserted into the organ sought to be examined and advanced a short distance while outer tube 9 is held stationary. After such advancement, the process is reversed, i.e. outer tube 9 is advanced a short distance while holding stationary inner tube 10. Thereafter, this process is repeated over and over again, advancing first one and then the other into the organ sought to be examined to the situs within the organ sought to be examined. The bell-shaped flaring distal portion 23 of the outer tube 9 tends to push the sidewalls of the organ away from and allows free advancement of inner tube 10, which then can be moved without hindrance at least to a position co-extensive with the terminal free mouth of the bell 23 of outer tube 9. The process is then repeated until the desired location within the organ to be examined is reached.

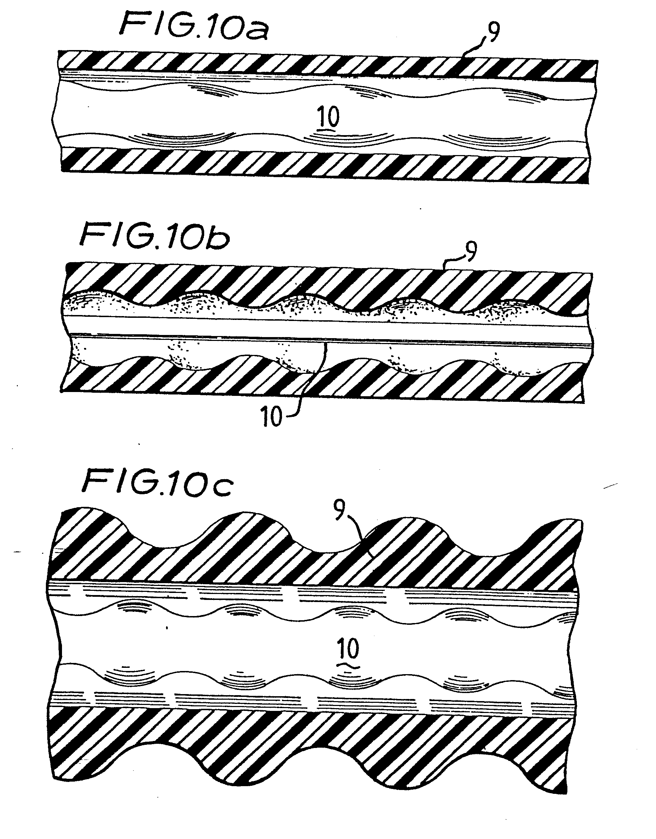

Friction is always an important consideration whenever one surface is moved across another. When a foreign body, such as outer tube 9 is inserted into an organ (for example, a colon) for examination, friction between outer tube 9 and the organ and outer

16.

tube 9 and inner tube 10 becomes a factor worthy of consideration. With respect to Figure 10(a), an embodiment is shown whereby inner tube 10 can be so constructed to have an undulating outer wall surface, the inner and outer wall surfaces of outer tube 9 being straight. Such a construction reduces friction between the outer wall surface of inner tube 10 and the inner wall surface of outer tube 9. With respect to Figure 10(b), another embodiment is shown whereby the inner wall surface of outer tube 9 is undulating and the outer wall surface of inner tube 10 is straight. Such a construction reduces friction between the two tubes, whenever they are moved relative to one another. In Figure 10(c) still another embodiment is shown whereby the outer wall surfaces of both tubes 9 and 10 are undulating. Such a construction not only reduces friction between the two tubes 9 and 10, but also between tube 9 and the sidewall of the organ undergoing examination. Various prior art lubricants may be used in that space delimited by the inner wall surface of outer tube 9 not otherwise occupied by inner tube 10 as well as on the outer wall surface of flexible tube 9. The undulating surfaces of tubes 9 and 10 (as shown in Figures 10(a), 10(b) and 10(c)) may be either longitudinal (not shown) or helical (as shown) and for

17.

best results face a smooth surface such as another tube or an organ sidewall.

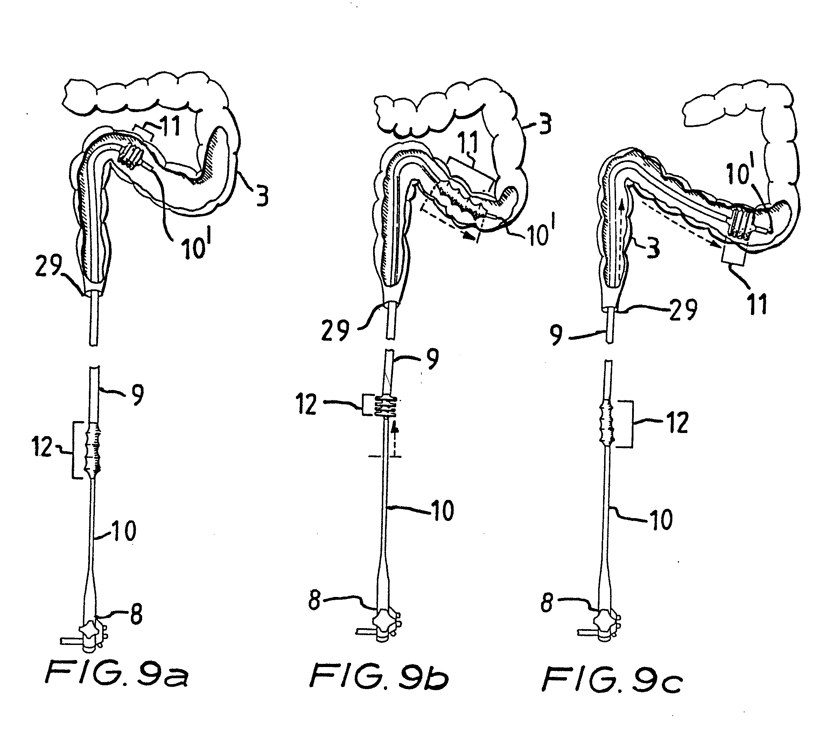

Associated with the description of the structure of each of the three embodiments of this invention, a brief description of the manner in which that embodiment was used in actual practice has been described and it will be noted that there is a procedure common to all embodiments, namely a movement of one tube a short distance, then the other coaxially sliding in or over the first tube and then a repetition of this process until the desired location is reached within the organ. Figures 9(a), 9(b) and 9(c) graphically represent the procedures thus described employing endoscope 7 of Figure 3. Element 29 represents the opening in an animal's body, in this instance, the anus of a human colon and endoscope 7 is shown therein as an example for fully understanding of the method of operation (use) of all the embodiments of the present invention; all of which are operated in essentially the same manner. It will be remembered that compression of distal bellows 11 requires an expansion of proximate bellows 12, as shown in Figure 9(a). The distal portion 10' of inner tube 10 is advanced through the colon a short distance, that short distance being delimited by the amount of laid distance that distal bellows 11 expands and/or proximate

0184399

18.

bellows 12 is contracted into a compressed state. See Figure 9(b). Operation of this particular embodiment starts from that state of the instrument shown in Figure 9(a) to that state shown in Figure 9(b) and is achieved by first holding stationary inner tube 10 and pushing outer tube 9 as far as it will go thereby compressing distal bellows 11 and simultaneously extending proximate bellows 12. Subsequently, while holding outer tube 9 stationary, inner tube 10 is then advanced as far as the expansion of distal bellows 11 and compression of proximate bellows 12 will permit thereby achieving the state as shown in Figure 9(b). The procedure is then reversed and the configuration of the bellows 11 and 12 and the position of distal means 10' is shown in Figure 9(c), which is the same as that shown in Figure 9(a) except for the advancement of distal portion 10' along the sigmoid portion 3 of the colon. This process is repeated over and over again to the desired location and it has been found that distal portion 10' will follow the curvilinear configurations of the colon to its deepest regions without requiring first straightening out any such loops or bends thereby permitting examination of those deep within the colon regions.

19.

CLAIMS:

1.     An endoscope (6,7,21,22) comprising a flexible tube (10) having a proximate end portion (27) in communication with a control unit (8), a body portion and a distal end portion (10'), the tube containing light transmitting means (20) whereby objects outside the free end of the distal end portion may be illuminated and viewed through the control unit CHARACTERISED IN THAT a further flexible (outer) tube (9) co-axially slideable about the body portion and the distal end portion (10') of the (inner) tube (10) is provided with means (11,31, 23) guarding the distal end of the inner tube whilst allowing relative incremental axial movements of the inner and outer tubes without the risk of tissue being trapped between them.

2.     An endoscope as claimed in claim 1 and FURTHER CHARACTERISED IN THAT the guard means comprise expansion and contraction means (11) so attached between the distal end (17) of the outer tube (9) and a point (16) near the distal end portion (10') of the inner tube (10) that the means can expand and contract axially of the inner and outer tubes.

3.     An endoscope as claimed in claim 2 and FURTHER CHARACTERISED IN THAT further expansion and contraction means (12) are attached between the proximate end (18) of the outer tube (9) and the proximate end portion (15) of the inner tube (10).

4.  An endoscope as claimed in claim 2 or claim 3 and FURTHER CHARACTERISED IN THAT the expansion and contraction means (11,12) consist of a tubular accordion or bellows-shaped member co-axially about the inner tube (10) and extending from an end of the outer tube (9).

5.  An endoscope as claimed in claim 3 or claim 4 and FURTHER CHARACTERISED IN THAT the axial distance by which both expansion and contraction means (11,12) may expand is essentially equal.

6.  An endoscope as claimed in any of claims 3 to 5 and FURTHER CHARACTERISED IN THAT the free space between the inner and outer tubes (10,9) and within the expansion and contraction means (11,12) is filled with a lubricant (19).

7.  An endoscope as claimed in claim 1 and FURTHER CHARACTERISED IN THAT the guard means (31) comprise a tubular shield (24) co-axially encompassing the distal end portion of the outer tube (9), the distal end (25) of the tubular shield being attached to the inner tube (10) adjacent the distal end portion (10') thereof; the outer tube being axially reciprocable between the inner tube and the tubular shield.

8.  An endoscope as claimed in claim 1 and FURTHER

21.

CHARACTERISED IN THAT the guard means comprise a radially outwardly flared, bell-shaped end (23) to the distal end portion of the outer tube (9).

9.      An endoscope as claimed in any of claims 1 to 8 and FURTHER CHARACTERISED IN THAT either the outer wall surface of the outer tube (9), the inner wall surface of the outer tube (9) or the outer wall surface of the inner tube (10), or the outer wall surfaces of the outer and inner tubes or the outer and inner wall surfaces of the outer tube is/are provided with undulations.

PRIOR ART

## FIG.1a

## FIG.1b

## FIG.1c

## FIG.1d

## FIG.1e

2/9

0184399

FIG.2

FIG.3

FIG.4a

FIG.4b

3/9

0184399

0184399

4/9

FIG.5

0184399

8

FIG.6b 23

9

10

10'

10

9

FIG.6a

22

9

6b

23

6b

10'

20

# FIG.7a

# FIG.7b

0184399

FIG.8a

FIG.8b

0184399

FIG. 9a    FIG. 9b    FIG. 9c

0184399

## FIG.10a

10

9

## FIG.10b

9

10

## FIG.10c

9

10